(19) Europäisches Patentamt
European Patent Office
Office européen des brevets

(11) Veröffentlichungsnummer: **0 009 688**
**B1**

(12) # EUROPÄISCHE PATENTSCHRIFT

(45) Veröffentlichungstag der Patentschrift: **02.03.83**

(51) Int. Cl.³: **A 61 K 47/00**, A 23 K 1/10

(21) Anmeldenummer: **79103441.6**

(22) Anmeldetag: **14.09.79**

(54) **Medikiertes Tierfutter auf Basis Lebermehl und Verfahren zu seiner Herstellung.**

(30) Priorität: **25.09.78 DE 2841668**

(43) Veröffentlichungstag der Anmeldung: **16.04.80 Patentblatt 80/8**

(45) Bekanntmachung des Hinweises auf die Patenterteilung: **02.03.83 Patentblatt 83/9**

(84) Benannte Vertragsstaaten: **AT BE CH DE FR GB IT NL**

(56) Entgegenhaltungen:
**DE-A-1 965 709**
**DE-A-2 325 036**
**FR-A-2 180 674**
**FR-A-2 265 289**
**US-A-3 862 336**

(73) Patentinhaber: **BAYER AG, Zentralbereich Patente, Marken und Lizenzen, D-5090 Leverkusen 1, Bayerwerk (DE)**

(72) Erfinder: **von Bittera, Miklos, Max-Scheler-Strasse 7, D-5090 Leverkusen 3 (DE)**
Erfinder: **Voege, Herbert, Dr., Martin-Buber-Strasse 41, D-5090 Leverkusen 3 (DE)**
Erfinder: **Bauditz, Reinwalt, Dr., In der Beek 28 B, D-5600 Wuppertal 1 (DE)**

**EP 0 009 688 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Medikiertes Tierfutter auf Basis Lebermehl und Verfahren zu seiner Herstellung

Die orale Verabreichung von Arzneimitteln bei Tieren ist, besonders wenn diese Arzneimittel einen unangenehmen, zumeist bitteren Geschmack besitzen, häufig mit großen Schwierigkeiten verbunden. Das oral, beispielsweise über ein medikiertes Futter, gegebene Arzneimittel wird häufig nicht akzeptiert und die gewünschte orale Behandlung kann entweder gar nicht oder nur im beschränkten und damit meist unzureichenden Maße unter Zwang durchgeführt werden.

Bei Nutztieren wie Rind und Schwein tritt die Behandlung mit medikiertem Futter in niedriger Dosierung über einen längeren Zeitraum mehr und mehr in den Vordergrund. Bei Haustieren wie Hund und Katze beschränken sich solche Futterzusätze in der Regel auf neutral schmeckende Zusatzstoffe zur Ernährung und Prophylaxe wie Vitamine, Spurenelemente und ähnliche, da unangenehm schmeckende Arzneimittel, insbesondere in der wirksamen Einmal-Dosis, von den Tieren nicht aufgenommen werden.

Es bestand daher ein stark ausgeprägtes Bedürfnis nach einer Arzneizubereitung für Tiere, insbesondere für Haustiere wie Hunde und Katzen, mit Hilfe welcher unangenehm, zumeist bitter schmeckende Arzneimittelwirkstoffe in problemloser und exakt dosierter Form oral appliziert werden können, d. h. freiwillig von den Tieren aufgenommen werden.

Es wurde nun gefunden, daß durch Einsatz von Lebermehl als Formulierungshilfsstoff (in medikiertem Tierfutter auf Basis Lebermehl) eine freiwillige orale Aufnahme des Arzneimittelwirkstoffs (»self-intake-Prinzip«) durch das zu behandelnde Tier ermöglicht wird.

Die Erfindung betrifft daher ein medikiertes Tierfutter auf der Basis Lebermehl, dessen zu verabreichende Trockenmasse 50–99 Gew.-% Lebermehl mit einem Gehalt an mindestens 65% Rohprotein, mindestens 42% fermentlöslichen Rohproteinen, maximal 18% Rohfett, maximal 2,5% Natriumchlorid und maximal 10% Wasser, 0,1–20 Gew.-Teile Wirkstoff, 1–20 Gew.-Teile Bindemittel und 0,01–5 Gew.-Teile weitere Hilfsstoffe enthält.

Unter Lebermehl ist ein Erzeugnis zu verstehen, daß durch Trocknen und Mahlen von frischer Leber warmblütiger Landtiere gewonnen wird und extrahiert sein kann (Siehe dazu H. J. Entel, N. Förster und E. Hinckers: Futtermittelrecht FMV-Anlage 1. Teil 1.1. Tie, Seite 41, Verlag P. Parey 1970/78, Stand: März 1978).

Es können für die Herstellung des erfindungsgemäßen medikierten Tierfutters auch Lebermehle eingesetzt werden, die von der oben angegebenen Norm geringfügig abweichen.

Als Formulierhilfsmittel wird extrahiertes Lebermehl bevorzugt.

Lebermehl ist als Hilfsstoff für Arzneipräparate bisher nicht eingesetzt worden, wohl aber als Futterzusatz. So ist ein extrahiertes Lebermehl unter dem Warenzeichen MURNIL® im Handel erhältlich. Als galenischer Hilfsstoff ist Lebermehl sonst auch denkbar ungeeignet, da es sehr hydrophob und elastisch ist.

Die Arzneiformulierung des Wirkstoffs bzw. Wirkstoffgemisches mit dem (vorzugsweise extrahierten und entfetteten) Lebermehl findet daher unter Zusatz eines in der Pharmazie üblichen Bindemittels statt. Als Bindemittel kommen für die Herstellung des medikierten Tierfutters auf Basis Lebermehl vorzugsweise in Frage: Stärke, Polyvinylpyrrolidon, Gelatine, Alkalimetall-Alginate, z. B. Natriumalginat sowie Cellulose-Derivate wie z. B. Methyl- und Carboxymethylcellulose. Die Auswahl des Bindemittels richtet sich in erster Linie nach der herzustellenden Formulierung (z. B. Keksform, Pellet-, Kugelform usw.). Im allgemeinen werden der zu verabreichende Wirkstoff (bzw. Wirkstoffgemisch) und das Lebermehl, unter Zusatz weiterer Hilfsstoffe, gemischt und mit einer vorzugsweise wäßrigen Lösung des pharmazeutisch üblichen Bindemittels geknetet. Die teigartige Masse kann auf die verschiedensten in der Lebensmittel- bzw. Pharmaindustrie üblichen Arten zur gebrauchsfertigen Arzneiform proportioniert werden, die dann von den Tieren freiwillig aufgenommen wird.

So kann die Masse beispielsweise über eine Strangpresse gegeben werden und der Strang so abgeschnitten werden, daß das Stück die Tierdosis des Arzneimittels darstellt. Es kann auch Gebäck aus der ausgewalzten Masse ausgestochen werden. Die Masse kann auch über einen beliebigen Granulator gegeben werden. Im nächsten Verfahrensschritt wird die Masse getrocknet — wobei die Trocknungstemperatur von der Thermostabilität des Wirkstoffes abhängt. Vorzugsweise wird bei 30 bis 180°C, insbesondere bei 80 bis 140°C, getrocknet. Dann wird in üblicher Weise verpackt, wobei das Granulat z. B. in Sachets, Tüten oder Becher als Einzeldosis abgefüllt wird.

Als weitere Hilfsstoffe können Stabilisatoren aller Art für den Wirkstoff eingesetzt werden, z. B. Antioxydantien, wie Butylhydroxyanisol, Natrium-meta-bisulfit, Konservierungsmittel, z. B. p-Hydroxybenzoesäure-ester, Sorbinsäure, Benzoesäure, aber auch Farbstoffe wie Eisenoxid-Pigmente und andere Lebensmittelfarbstoffe, zusätzlich Aromen wie Fleisch-, Leber-Braten-Aroma.

Als Wirkstoffe kommen praktisch alle in der Veterinärmedizin einsetzbaren Wirkstoffe in Betracht, vorzugsweise werden unangenehm schmeckende Wirkstoffe eingesetzt, die einen meist bitteren Geschmack aufweisen, beispielhaft seien genannt: Anthelmintika wie Praziquantel, Tetramisol, Levamisol, Niclosamid, Piperazinsalze, Antibiotika, wie Chloramphenicol, Tetracyclin, Penicillin-Derivate wie das Benzathin-Salz des Penicillin G, Ampicillin,

Cloxacillin, Hormone, z. B. 6-Chlor-6-dehydro-17α-acetoxyprogesteron u. a.

Bei der Herstellung des medikierten Tierfutters werden vorzugsweise folgende Teilmengen der oben angegebenen Ingredienzien zusammengegeben, wobei die folgenden Zahlenangaben sich auf Gewichtsteile beziehen:

Lebermehl 50 bis 99,
vorzugsweise 85 bis 98,
Wirkstoff (bzw. Wirkstoffgemisch) 0,1 bis 20,
vorzugsweise 0,5 bis 10,
Bindemittel 1 bis 20,
vorzugsweise 2 bis 10,
weitere Hilfsstoffe 0,01 bis 5,
vorzugsweise 0,1 bis 2.

Auf 100 Gew.-Teile des obigen Feststoffgemisches werden 10 bis 60, vorzugsweise 30 bis 50 Gew.-Teile Wasser gegeben, welches nach dem Anteigen wieder weggetrocknet wird.

Ein weiterer Gegenstand der Erfindung ist ein Verfahren zur Herstellung von medikiertem Tierfutter, das darin besteht, daß man 100 Gew.-Teile eines Gemisches, bestehend aus 50 bis 99 Gew.-Teilen Lebermehl mit einem Gehalt an mindestens 65% Rohprotein, mindestens 42% fermentlöslichen Rohproteinen, maximal 18% Rohfett, maximal 2,5% Natriumchlorid und maximal 10% Wasser, 0,1 bis 20 Gew.-Teilen Wirkstoff, 1 bis 20 Gew.-Teilen Bindemittel, 0,01 bis 5 Gew.-Teilen weiteren Hilfsstoffen mit 1 bis 60 Gew.-Teilen Wasser vermischt, die entstehende teigige Masse nach an sich bekannten Methoden proportioniert und trocknet.

In der fertigen erfindungsgemäßen Lebermehlpräparation (medikiertes Tierfutter auf Basis Lebermehl) sind die Bestandteile dann vorzugsweise in den oben angegebenen Bereichen vorhanden.

Durch das erfindungsgemäße Verfahren zur Herstellung des medikierten Tierfutters auf Basis Lebermehl ist es möglich, durch entsprechende Zumischung des zu verabreichenden Wirkstoffs und Proportionierung der zu verabreichenden Arzneimittelform eine exakte Dosierung des Arzneimittels zu realisieren, so daß in Abhängigkeit vom Gewicht des zu behandelnden Tieres eine exakte Menge des medikierten Futters gegeben werden kann. Siehe dazu auch die in den nachfolgenden Beispielen angegebenen Individualdosierungen bezüglich einiger Wirkstoffe.

Beispiel 1

980,0 g entfettetes Lebermehl werden mit 10,0 g Praziquantel-Wirkstoff der Formel

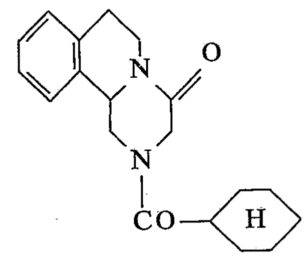

gemischt.

Die Mischung wird mit einer Lösung von 10,0 g Polyvinylpyrrolidon 25 in 500,0 g Wasser zu einem Teig geknetet.

Der Teig wird in herkömmlicher Weise ausgerollt und davon Stücke abgetrennt, die nach dem Trocknen 5 g schwer sind und somit 50 mg Praziquantel-Wirkstoff enthalten. (Dies entspricht einer wirksamen Dosis für einen 10 kg schweren Hund.) Das Trocknen der Teigstücke geschieht im Trockenschrank bei 50° C.

Beispiel 2

Zehn Hunden (Körpergewicht 8,5 bis 10,2 kg) wurden jeweils eine herkömmliche 50 mg Praziquantel enthaltende Tablette, ein nach Beispiel 1 hergestelltes 50-mg-Praziquantel-Lebermehl-Präparat und eine herkömmliche 50-mg-Praziquantel-Tablette, die mit Leberaroma aromatisiert war, angeboten.

Während das nach Beispiel 1 hergestellte Präparat von jedem Hund akzeptiert und gefressen wurde, wurden die herkömmlichen Tabletten sowie die mit Leberaroma aromatisierten herkömmlichen Tabletten von den Tieren nicht aufgenommen.

Beispiel 3

1000 g entfettetes Lebermehl werden mit 7 g Praziquantel-Wirkstoff gemischt und die Mischung mit 340 g eines 5,2%igen wäßrigen Natriumalginat-Schleims angefeuchtet und homogen vermischt.

Die Mischung wird dann über eine Futtermittel-Pelletier-Anlage im Labormaßstab gegeben und es werden Pellets mit einem Durchmesser von 4 mm hergestellt. Die Pellets werden im Trockenschrank bei 50° C 4 Stunden getrocknet und anschließend abgefüllt.

Anschließend wurde ein Akzeptanztest bei 7 Hunden (durchschnittliches Gewicht ca. 10 kg) durchgeführt. Alle Hunde akzeptierten die gemäß Beispiel 3 hergestellten Pellets.

Beispiel 4

500 g entfettetes Lebermehl werden mit 10 g Levamisol-Hydrochlorid (L-2,3,5,6-Tetrahydro-6-phenyl-imidazo-[2,1-b]thiazol-hydrochlorid) und 0,5 g Natriummetabisulfit (Antioxydans) ge-

mischt und die Mischung mit 170 g eines 5,2%igen wäßrigen Natriumalginat-Schleims angefeuchtet und homogen vermischt.

Die Mischung wird dann wie in Beispiel 3 beschrieben zu Pellets (Durchmesser: 4 mm) aufgearbeitet.

Von je 10 Hunden verschiedener Rasse, denen im Markt befindliche Levamisol-Hydrochlorid-Tabletten, pharmazeutisches Levamisol-Hydrochlorid enthaltendes Granulat auf Basis Glucose sowie nach Beispiel 4 hergestellte Lebermehl-Pellets angeboten wurden, nahmen 9 das nach Beispiel 4 hergestellte Medikament auf, während in den anderen beiden Gruppen lediglich ein Hund eine Tablette aufnahm, keiner jedoch das Granulat auf Basis Glucose.

Beispiel 5

A) 950,0 g Lebermehl werden mit 10,0 g Praziquantel-Wirkstoff homogen vermischt.
B) 30,0 g Polyvinylpyrrolidon 25 werden zusammen mit 10,0 g Lecithin in 500,0 g Wasser aufgelöst.

Die homogene Mischung nach A) wird mit der Lösung nach B) zu einem Teig geknetet.

Der Teig wird, wie in Beispiel 1 beschrieben, ausgerollt und davon Stücke abgetrennt, die nach dem Trocknen 5 g Praziquantel enthalten.

Die Präparation nach Beispiel 5 wurde 10 Hunden vorgelegt und von allen aufgenommen.

Beispiel 6

990 g entfettetes Lebermehl werden mit 5,0 g Chloramphenicol — einem unangenehm bitter schmeckenden Wirkstoff — homogen vermischt.

Die Mischung wird mit einer Lösung von 10,0 g Polyvinylpyrrolidon 25 in 500,0 g Wasser zu einem Teig geknetet und — wie bei Beispiel 1 beschrieben — zu Keksen verarbeitet. Pro 5 kg Körpergewicht des Hundes wurden 10 g Lebermehl-Keks nach Beispiel 6 angeboten. Von insgesamt 10 Hunden nahmen 8 die Kekse auf. Ein zum Vergleich angebotenes Stück eines handelsüblichen Chloramphenicol-Bolus wurde dagegen abgelehnt.

Beispiel 7

10,0 g Praziquantel-Wirkstoff, 20,0 g lösliche Stärke und 970,0 g entfettetes Lebermehl werden mit 300 ml Wasser angefeuchtet und gut durchgeknetet. Die so erhaltene Masse wird über eine Raspel 5 mm grob zerkleinert und schonend 5 Stunden lang bei 50°C im Trockenschrank getrocknet.

Das erhaltene Granulat wird 10 Hunden in Portionen von 5,0 g Granulat pro 10 kg Hundegewicht vorgelegt. 10 anderen Hunden wurde eine entsprechende Anzahl herkömmlicher, mit Milchzucker und Stärke granulierter Tabletten angeboten.

Das Lebermehl-Granulat gemäß Beispiel 7 wurde von 9 Tieren aufgenommen, die Tabletten von keinem Tier.

**Patentansprüche**

1. Medikiertes Tierfutter auf Basis Lebermehl, dessen zu verabreichende Trockenmasse

50 bis 99 Gewichtsteile Lebermehl mit einem Gehalt an mindestens 65% Rohprotein, mindestens 42% fermentlöslichen Rohproteinen, maximal 18% Rohfett, maximal 2,5% Natriumchlorid und maximal 10% Wasser,
0,1 bis 20 Gewichtsteile Wirkstoff
1 bis 20 Gewichtsteile Bindemittel
0,01 bis 5 Gewichtsteile weitere Hilfsstoffe

enthält.

2. Medikiertes Tierfutter gemäß Anspruch 1, dadurch gekennzeichnet, daß dessen zu verabreichende Trockenmasse

85 bis 98 Gewichtsteile Lebermehl mit einem Gehalt an mindestens 65% Rohprotein, mindestens 42% fermentlöslichen Rohproteinen, maximal 18% Rohfett, maximal 2,5% Natriumchlorid und maximal 10% Wasser,
0,5 bis 10 Gewichtsteile Wirkstoff
1 bis 10 Gewichtsteile Bindemittel
0,1 bis 2 Gewichtsteile weitere Hilfsstoffe

enthält.

3. Medikiertes Tierfutter gemäß Anspruch 1, dadurch gekennzeichnet, daß es sich beim Wirkstoff um einen in der Veterinärmedizin üblichen Wirkstoff und bei dem Bindemittel um ein Bindemittel der Reihe:

Stärke, Polyvinylpyrrolidon, Gelatine, Natriumalginat, Cellulose-Derivat handelt.

4. Verfahren zur Herstellung von medikiertem Tierfutter, dadurch gekennzeichnet, daß man 100 Gew.-Teile eines Gemisches bestehend aus

50 bis 99 Gewichtsteilen Lebermehl mit einem Gehalt an mindestens 65% Rohprotein, mindestens 42% fermentlöslichen Rohproteinen, maximal 18% Rohfett, maximal 2,5% Natriumchlorid und maximal 10% Wasser,
0,1 bis 20 Gewichtsteilen Wirkstoff
1 bis 20 Gewichtsteilen Bindemittel
0,01 bis 5 Gewichtsteilen weiteren Hilfsstoffen

mit 10 bis 60 Gewichtsteilen Wasser vermischt, die entstehende teigige Masse nach an sich bekannten Methoden proportioniert und trocknet.

## Claims

1. Medicated animal feed based on liver meal, the dry mass of which to be administered contains

50 to 99 parts by weight of liver meal with a content of at least 65% of raw protein, at least 42% of fermentsoluble raw proteins, at most 18% of raw fat, at most 2.5% of sodium chloride and at most 10% of water,
0.1 to 20 parts by weight of active compound
1 to 20 parts by weight of binder and
0.01 to 5 parts by weight of other auxiliaries.

2. Medicated animal feed according to Claim 1, characterised in that the dry mass of which to be administered contains

85 to 98 parts by weight of liver meal with a content of at least 65% of raw protein, at least 42% of fermentsoluble raw proteins, at most 18% of raw fat, at most 2.5% of sodium chloride and at most 10% of water,
0.5 to 10 parts by weight of active compound
2 to 10 parts by weight of binder and
0.1 to 2 parts by weight of other auxiliaries.

3. Medicated animal feed according to Claim 1, characterised in that the active compound is an active compound customary in veterinary medicine and the binder is a binder from the series:

starch, polyvinylpyrrolidone, gelatin, sodium alginate and a cellulose derivative.

4. Process for the preparation of medicated animal feed, characterised in that 100 parts by weight of a mixture consisting of

50 to 99 parts by weight of liver meal with a content of at least 65% of raw protein, at least 42% of fermentsoluble raw proteins, at most 18% of raw fat, at most 2.5% of sodium chloride and at most 10% of water,
0.1 to 20 parts by weight of active compound
1 to 20 parts by weight of binder and
0.01 to 5 parts by weight of other auxiliaries

are mixed with 10 to 60 parts by weight of water and the pasty mass formed is proportioned and dried by methods which are in themselves known.

## Revendications

1. Aliment médicamenté pour animaux à base de farine de foie dont la masse sèche à administrer contient

50 à 99 parties en poids de farine de foie ayant une teneur d'au moins 65% en protéine brute, au moins 42% de protéines brutes solubles dans les ferments, 18% maximum de graisse brute, 2,5% maximum de chlorure de sodium et 10% maximum d'eau, .
0,1 à 20 parties en poids d'une substance active,
1 à 20 parties en poids d'un agent liant et
0,01 à 5 parties en poids d'autres substances auxiliaires.

2. Aliment médicamenté pour animaux suivant la revendication 1, caractérisé en ce que sa masse sèche à administrer contient:

85 à 98 parties en poids de farine de foie ayant une teneur d'au moins 65% de protéine brute, au moins 42% de protéines brutes solubles dans les ferments, 18% maximum de graisse brute, 2,5% maximum de chlorure de sodium et 10% maximum d'eau,
0,5 à 10 parties en poids d'une substance active,
2 à 10 parties en poids d'un agent liant,
0,1 à 2 parties en poids d'autres substances auxiliaires.

3. Aliment médicamenté pour animaux suivant la revendication 1, caractérisé en ce que, en ce qui concerne la substance active, il s'agit d'une substance active habituellement utilisée en médecine vétérinaire tandis que, en ce qui concerne l'agent liant, il s'agit d'un agent liant de la série:

amidon, polyvinylpyrrolidone, gélatine, alginate de sodium, dérivé de la cellulose.

4. Procédé de préparation d'un aliment médicamenté pour animaux, caractérisé en ce qu'on mélange 100 parties en poids d'un mélange constitué de

50 à 99 parties en poids de farine de foie ayant une teneur d'au moins 65% en protéine brute, au moins 42% de protéines brutes solubles dans les ferments, 18% maximum de graisse brute, 2,5% maximum de chlorure de sodium et 10% maximum d'eau,
0,1 à 20 parties en poids d'une substance active,
1 à 20 parties en poids d'un agent liant,
0,01 à 5 parties en poids d'autres substances auxiliaires,

avec 10 à 60 parties en poids d'eau, puis on proportionne et sèche la masse pâteuse obtenue conformément à des méthodes connues en soi.